# FASCICULE DE BREVET EUROPEEN

(11) **EP 1 117 346 B1**
(45) Date de publication et mention de la délivrance du brevet: **15.02.2006**
(21) Numéro de dépôt: 00956645.6
(22) Date de dépôt: 09.08.2000
(51) Int. Cl.: A61F 2/06, A61F 2/01, A61B 17/22

(54) **DISPOSITIF INTRALUMINAL EXPANSIBLE**
AUSDEHNBARE INTRALUMINALE VORRICHTUNG
EXPANDABLE INTRALUMINAL DEVICE

(30) Priorité: 10.08.1999 FR 9910362
(43) Date de publication de la demande: 25.07.2001
(73) Titulaire: B. Braun Medical Société Anonyme, 92100 Boulogne Billancourt (FR)
(72) Inventeur: FORBER, Simon, John, F-86170 Yversay (FR)
(74) Mandataire: Lerner, François
(86) Numéro de dépôt international: PCT/FR2000/002283
(87) Numéro de publication internationale: WO 2001/010346

(56) Documents cités:
- EP-A- 0 533 511
- EP-A- 0 925 763
- WO-A-98/33443
- WO-A-98/39053
- WO-A-98/51237
- WO-A-99/44510
- WO-A-99/44542
- US-A- 5 695 519
- US-A- 5 814 064
- US-A- 5 925 060

## Description

L'invention concerne un dispositif intraluminal adapté pour être introduit dans un conduit corporel (en particulier un vaisseau sanguin) pour retenir d'éventuels débris (vasculaires) pouvant être présents dans le fluide corporel (sang).

Dans ce domaine, on connaît en particulier les filtres sanguins (en particulier les filtres temporaires prévus pour être introduits dans un vaisseau pour une durée comprise entre quelques heures et environ quinze jours), comme par exemple décrit dans WO-A-99/44510, ou les dispositifs d'angioplastie, comme par exemple décrits dans WO-A-98/09683, ou encore les systèmes utilisés pour capturer ou retenir des "matériaux emboliques" pendant une procédure d'artériectomie ou de thrombectomie (comme dans WO-A-99/44542).

A noter qu'éventuellement, le dispositif qui va être présenté ci-après pourrait même constituer un implant vasculaire prévu pour être définitivement mis en place dans un vaisseau, voire un autre conduit corporel (urètre, ....), en étant amovible de la "tige", c'est-à-dire de l'élément allongé qui permet de le manoeuvrer ou de le guider jusqu'à son endroit d'implantation dans le conduit corporel considéré, depuis l'extérieur du corps du patient (à la manière, par exemple, de la liaison amovible qui est divulguée dans EP-A-925 763).

Les domaines d'application du dispositif de l'invention sont donc variés.

Dans le domaine vasculaire, un objet de l'invention est de proposer un dispositif intraluminal qui puisse donc en particulier retenir d'éventuels débris vasculaires pouvant être présents dans le sang, ceci d'une manière particulièrement fiable, avec un dispositif facile à utiliser, à mettre en place, voire si nécessaire à retirer, et qui puisse être fabriqué en série, dans des conditions de fabrication performantes.

L'invention s'est en outre attachée à proposer un tel dispositif intraluminal que l'on puisse aisément introduire dans un état radialement resserré, à l'intérieur du conduit dans lequel le dispositif va ensuite être déployé.

Pour satisfaire ces objectifs, le dispositif selon l'invention est tel que défini par la revendication 1. Des modes de réalisation avantageux sont définis par les sous-revendications 2 à 10.

Le document EP-A-0 533 511 est considéré comme étant l'état de la technique le plus proche.

Dans certains cas, les dispositifs existants qui doivent s'expanser radialement pour occuper leur position opératoire, s'ouvrent difficilement ou non régulièrement sur leur périphérie, ce qui peut provoquer un mauvais alignement axial du dispositif dans son conduit récepteur ou, plus grave encore, une mauvaise ouverture, voire entraîner un défaut d'efficacité.

Pour améliorer la fiabilité d'ouverture radiale du dispositif, l'invention conseille que le film équipant la structure radialement expansible s'étende sur les côtés proximal et distal en y présentant plusieurs orifices, par groupes, à différentes distances radiales par rapport à l'axe, dans l'état radialement déployé de la structure.

Comme indiqué ci-avant, le dispositif de l'invention est prévu pour ne pas constituer un obstacle à la circulation du sang (ou du fluide corporel correspondant) dans le conduit d'implantation (à la différence des "dispositifs occlusifs" connus, comme par exemple dans WO-A-98/42059, ou US-A-5 925 060.

Or, à nouveau pour des raisons de performance dans l'ouverture radiale du dispositif et/ ou pour potentiellement permettre au dispositif de former un filtre intraluminal en piégeant des débris à l'intérieur de sa structure radialement expansible, l'invention propose que le film de la structure radialement expansible, non seulement s'étende sur les côtés proximal et distal de cette structure, mais y présente plusieurs orifices pour le passage au moins du sang, les orifices étant, par groupes, de tailles différentes du côté proximal.

En relation avec ce qui précède, et pour accroître la performance, il est même conseillé dans l'invention que, du côté proximal, les orifices de plus petite taille soient plus près de la zone intermédiaire et plus éloignés de l'axe, dans l'état radialement déployé de la structure, que les orifices de plus grande taille.

En particulier dans le cas où le dispositif de l'invention serait tel que sa structure radialement expansible pourrait être réalisée afin d'être amovible par rapport à sa "tige " de manoeuvre (ladite structure pouvant alors être utilisée en particulier en tant que dispositif d'occlusion "perméable", c'est-à-dire laissant passer le liquide corporel du conduit considéré), un objet complémentaire de l'invention concerne la forme privilégiée que pourrait alors prendre la structure radialement expansible afin de renforcer encore la capacité du dispositif à satisfaire aux premières exigences citées en début de description.

A cet effet, il est prévu que, dans ledit état radialement déployé de la structure, le treillis de fils présente une forme générale cylindrique.

Dans ces conditions, une caractéristique complémentaire de l'invention conseille que :
- les fils du treillis soient réunis entre eux et maintenus par des éléments de cerclage, auxdites extrémités proximale et distale de la structure,
- le film présente une forme comparable à celle du treillis, en étant resserré vers lesdites extrémités proximale et distale de la structure,
- le film soit disposé de façon lâche autour du treillis de fils, sans y être fixé, et
- le film soit fixé au moins à l'élément de cerclage distal.

Dans une application du filtre, mais plus généralement dans toute application nécessitant une large capacité de pénétration du flux de fluide, de possibles débris en circulation, ou autres ... à l'intérieur de la structure radialement expansible, une autre caractéristique de l'invention prévoit que, du côté proximal, mais à distance de l'extrémité proximale de la structure, les fils du treillis sont rapprochés les uns des autres par séries, pour former des torons définissant entre eux des ouvertures de taille supérieure à celle des mailles du treillis, pour laisser pénétrer dans ce treillis des débris vasculaires.

En outre, une caractéristique complémentaire de l'invention conseille que les torons de fils soient alors réunis entre eux à l'extrémité proximale de la structure où qu'ils soient fixés à l'élément de cerclage proximal, à l'endroit duquel la structure est en outre liée à la tige.

Dans ce cas, les torons joueront en quelque sorte le rôle de "anse" permettant à la fois de définir les ouvertures d'entrée à l'intérieur du treillis de fils et de retenue de cette zone de filtration aval par rapport à la tige de manoeuvre située plus en amont du flux de fluide (lorsque le dispositif est installé dans son conduit corporel).

Pour favoriser encore les conditions de fabrication du dispositif, tout en préservant la fiabilité du dispositif, en particulier en termes de retenue des débris corporels, une caractéristique complémentaire de l'invention conseille, que du côté proximal, le film s'étende sensiblement suivant le contour de la limite périphérique entre la zone des fils en treillis et la zone des fils en torons.

Dans ce cas, on comprend que le film ne débuterait qu'à l'endroit de la base des torons, c'est-à-dire, en pratique (lorsque la structure expansible est en place dans son conduit corporel) sensiblement à la limite entre le côté proximal et la zone intermédiaire de plus large diamètre.

A cet égard, une caractéristique également complémentaire de l'invention conseille d'ailleurs que :
- les torons de fils s'étendent sur le côté proximal de la structure, et
- le film s'étende exclusivement en zone intermédiaire et du côté distal de la structure, de sorte que le dispositif définit un filtre sanguin intravasculaire.
- les figures 1 et 2 montrent schématiquement un dispositif similaire (figure 1) et de l'ensemble tige/structure radialement expansible (figure 2), respectivement dans un état radialement resserré et radialement déployé de ladite structure ;
- la figure 3 montre en vue schématique partielle une alternative de réalisation de la structure expansible de la figure 2;
- la figure 4 montre schématiquement un premier mode de réalisation du dispositif et de l'ensemble tige/structure, dans une application filtre ;
- la figure 5 est une vue schématique en perspective d'un ensemble tige/ structure, comme sur la figure 4 ;
- la figure 6 est une coupe schématique selon la ligne VI-VI de la figure 4 ;
- la figure 7 montre schématiquement l'implantation d'un dispositif d'angioplastie, avec à son extrémité distale, une structure radialement expansible conforme à l'invention ;
- et les figures 8 et 9 montrent deux étapes opératoires pour la réalisation de l'intervention intraluminale concernée avec un dispositif selon la figure 1.

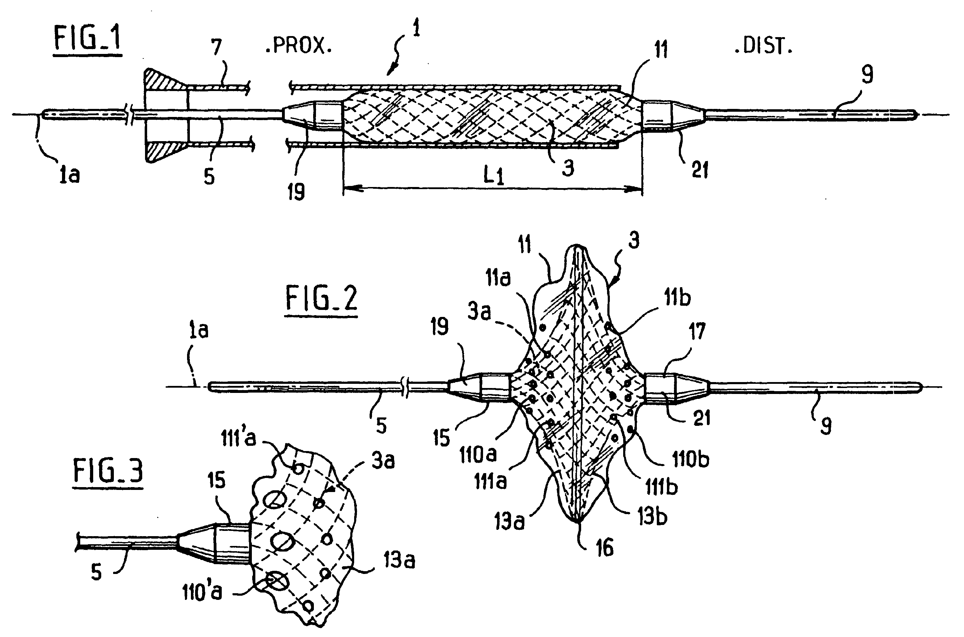

Sur la figure 1 tout d'abord, le repère 1 se rapporte à un dispositif intraluminal.

Le dispositif 1 comprend essentiellement une structure radialement expansible 3, disposée à une extrémité d'une tige de guidage 5, les éléments 3 et 5 ayant été glissés à l'intérieur d'un manchon tubulaire 7.

Le dispositif 1 présente un axe principal d'allongement 1a suivant lequel l'ensemble 3, 5, coulisse à l'intérieur de la gaine d'introduction 7.

Avantageusement, la tige 5 peut être un guide-fil (guide wire) métallique d'environ 0,2 mm à 0,9 mm de section.

On notera que sur certaines des figures, on a repéré PROX l'extrémité (ou le côté) proximal du dispositif et DIST, l'extrémité ou côté distal. Le côté proximal est le côté du dispositif le plus proche de l'endroit du corps par où le dispositif va être introduit dans le conduit à traiter, tandis que le côté distal est celui qui pénètre le plus profondément à l'intérieur dudit conduit. Ainsi, la structure radialement expansible 3 est située à l'extrémité distale de la tige de guidage 5, et à son extrémité distale, cette même structure 3 est prolongée par un prolongement de protection 9, globalement encore plus flexible que la tige 5 et de section comparable, servant à faciliter le passage de l'ensemble 3 - 5 à l'intérieur du conduit corporel.

Comme on le voit en comparant les figures 1 et 2, la structure expansible 3 est adaptée pour occuper, radialement à l'axe 1a, deux états extrêmes : un premier état radialement resserré et une première longueur L₁ comme sur la figure 1, et un second état radialement déployé, avec un second diamètre D₂ supérieur au premier diamètre et une seconde longueur axiale inférieure à la première longueur L₁.

Plus nettement sur la figure 2, on constatera en outre que la structure 3 comprend une armature constituée par un treillis de fils 3a face auxquels s'étend un film 11. Le film double extérieurement le treillis de fils dont il est structurellement distinct.

Le treillis de fils 3a définit un maillage et peut être réalisé comme décrit en colonne 3, ligne 15 - colonne 4, ligne 18 de US-A-5 733 294, avec pour particularité que la forme finale du treillis de fils est ici en disque, dans l'état au repos, sans contrainte radiale ou axiale, du treillis. Cette forme a pu être figée par action thermique. Les fils du treillis peuvent être en alliage nickel-titane (appelé "Nitinol®") ou en polymère à mémoire de forme, voire en acier inoxydable.

Comme illustré sur la figure 2, et en particulier pour une application en tant qu'élément distal dans un dispositif d'angioplastie, on peut réaliser le treillis 3a avec un maillage présentant, dans l'état radialement déployé de la structure, des côtés proximal et distal 13a, 13b, de forme extérieurement concave, suivant sensiblement deux hyperboles se raccordant entre elles suivant une arête sensiblement centrale 16 définissant un cercle s'étendant suivant un plan perpendiculaire à l'axe 1a, en zone intermédiaire (en l'espèce médiane) de la structure, là où cette dernière présente son plus grand diamètre D₂).

On notera que, dans l'hypothèse où la liaison entre la structure 3 et la tige 5 serait amovible, une telle structure en forme de disque (au repos) pourrait notamment être utilisée dans le cadre de l'application présentée dans US-A-5 733 294 (colonne 1, lignes 6 à 10), c'est-à-dire un dispositif vaso-occlusif en radiologique ou embolothérapie, notamment, étant toutefois précisé qu'il s'agirait alors d'une occlusion "sélective" dans la mesure où le film 11 présente des orifices pour un libre passage du fluide corporel du conduit d'implantation. Un diamètre de 100 à 200 microns doit convenir.

A cet égard, on voit plus clairement sur la figure 2 que le film 11 présente deux séries d'orifices, respectivement proximaux 11a et distaux 11b, dès lors que ce film recouvre dans l'exemple entièrement le treillis de film, sur toute sa surface.

Toujours dans l'exemple, chacune des deux séries de trous proximaux et distaux 11a, 11b, est scindée en deux groupes, 110a, 111a ; 110b, 111b. Chacun des groupes de trous est réparti circonférentiellement autour de l'axe 1a, symétriquement entre le côté proximal et le côté distal.

D'un même côté, les groupes de trous sont chacun situés à une distance radiale de l'axe différente de celle de l'autre groupe, ceci bien entendu dans l'état radialement déployé de la structure. Ainsi, les trous des groupes 110a, 120b, sont situés plus près de l'axe 1a que les trous des groupes 111a, 111b. Les premiers sont également plus près des extrémités respectivement distale et proximale de la structure que les seconds. Ceci participe à la fois à une circulation favorable du fluide corporel à travers la structure 3 et à une ouverture radiale favorable de cette structure. En effet, en pénétrant à l'intérieur du film 11, le fluide va avoir tendance à au moins temporairement "gonfler" la structure.

Le film 11 peut être un film en polyéthylène déposé par vaporisation autour du treillis de fils ou encore mis en place par l'intermédiaire d'un support cylindrique d'étirement, comme décrit dans EP-A-0 947 168.

De préférence, le film 11 sera disposé de façon lâche autour du treillis de fils 3a, c'est-à-dire que le film n'épousera pas la forme "en disque" du treillis, comme schématisé sur la figure 2.

Aux extrémités proximale et distale de la structure 3 (repérées respectivement 15 et 17 sur la figure 2), les fils du treillis 3 sont rapprochés les uns des autres et fixés à (interposés entre) deux bagues concentriques réunies ensemble pour former un cerclage de maintien proximal 19 et un cerclage de maintien distal 21. Ces cerclages sont respectivement eux-mêmes fixés à la tige de manoeuvre 5 et à l'embout d'orientation 9. De préférence, il s'agit d'anneaux radio-opaques (métalliques).

Dans l'exemple présenté, le treillis de fils 3 est donc fabriqué de manière à être radialement auto-expansible, c'est-à-dire qu'une fois sorti de sa gaine 7 dans laquelle il est radialement étroitement contraint dans un état sensiblement tubulaire (voir figure 1), il se déploie de lui-même, naturellement, jusque dans son état de la figure 2, par un effet de rappel "élastique" des fils.

Eventuellement, en alternative, si la tige 5 était un cathéter médical, un fin câble de contrôle pourrait être glissé à l'intérieur du cathéter pour traverser le cerclage double 19 ainsi que le maillage 3a, pour être fixé au cerclage double distal 21, permettant ainsi au treillis 3a d'être déplacé entre ses positions radialement contrainte et radialement déployée, par traction ou poussée sur ce fin guide.

Pour une possible intervention profonde, la tige 5 peut présenter une longueur comprise entre 120 cm et 180 cm.

L'introduction corporelle du dispositif 1 peut s'effectuer après une incision transcutanée, par la méthode dite de SELDINGER, et la structure 3, par exemple, peut être utilisée comme élément distal d'un dispositif d'angioplastie que l'on décrira ci-après.

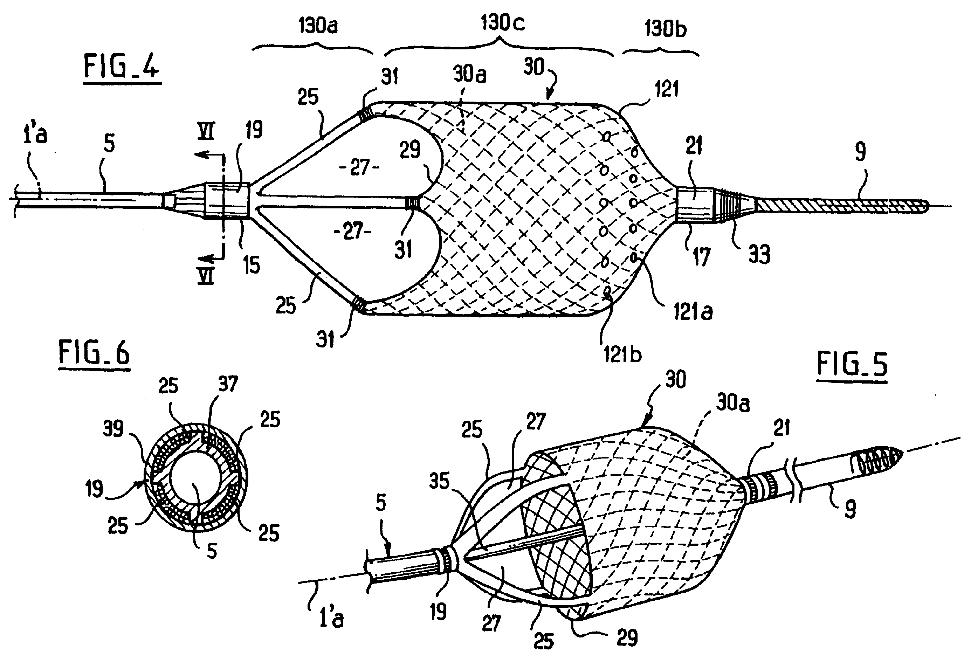

Intéressons-nous maintenant aux figures 4 et 5 pour décrire une alternative de réalisation.

Sur la figure 3, le film 11 est traversé, côté 13a par deux séries d'orifices 110'a, 111'a, de tailles différentes.

Les orifices les plus gros 110'a sont plus près de l'axe et de l'extrémité proximale que ceux de plus petite section.

Les premiers 110'a peuvent laisser les débris corporels (véhiculés par le liquide corporel en circulation) entrer dans le panier défini par le treillis de fils où ils seront piégés. Les deux séries d'orifices favorisent l'ouverture du panier 3a.

Sur la figure 4, la structure 3 a été remplacée par une structure 30 que l'on voit là dans son état radialement déployé, pleinement, c'est-à-dire sans aucune contrainte radiale exercée sur elle.

Dans cet état, on peut remarquer que la structure 30 est plus allongée axialement (axe 1'a) que la structure 3 de la figure 2. Par contre, on retrouve toujours la tige flexible de manoeuvre 5, l'embout distal de guidage 9 et les cerclages doubles, respectivement proximal et distal,19, 21.

Egalement comme déjà décrit, la structure 30 comprend un treillis de fils 30a doublé (en particulier recouvert) par un film 121.

Le film 121 peut en particulier être en polyéthylène (donc étanche au sang) ou éventuellement en polyuréthanne (trame pouvant ou non laisser passer le sang).

Si le film 121 n'est pas poreux au sang, des orifices tels que 121a, 121b, sont alors formés dans la zone distale 130b de la structure. De préférence, au moins deux séries d'orifices pour l'écoulement du fluide corporel seront prévus, avec une disposition axiale et radiale commune avec celle des séries d'orifices 110b, 111b, de la figure 2.

On remarquera que le film 121 ne s'étend pas sur toute la surface de la structure 30.

En effet, le treillis de fils 30a, qui peut être radialement auto-expansible, s'interrompt sensiblement à la limite entre la zone proximale 130a et la zone intermédiaire 130c de la structure, la séparation entre les zones apparaissant au changement de pentes : les deux zones proximale et distale forment des surfaces sensiblement coniques ou tronconiques (du moins orientées de biais par rapport à l'axe 1'a), tandis que la zone intermédiaire 130c pourra avantageusement présenter une surface tubulaire sensiblement cylindrique. Sa longueur pourra être au moins double de celle de chacune des zones 130a, 130b, ceci pour un contact étendu avec la surface intérieure de la paroi du conduit à traiter et pour une qualité de centrage, en particulier en zone tortueuse.

En zone proximale 130a, le treillis de fils 30a est tel que les fils du treillis sont rapprochés les uns des autres par séries, pour former des torons (en l'espèce, au nombre de quatre, voir également figure 4) repérés 25. Ces torons définissent deux à deux, entre eux, des ouvertures 27 de taille supérieure à celle des mailles du treillis, pour laisser pénétrer dans ce treillis des débris corporels véhiculés par le fluide concerné. Ainsi, la structure 30 peut constituer un filtre sanguin intravasculaire.

Le film 121, qui peut en l'espèce épouser sensiblement le contour extérieur de la zone en treillis 30a peut être formé par vaporisation d'un produit synthétique autour de ce treillis, de telle sorte que ce film s'arrête sensiblement suivant le contour de la limite périphérique, repérée 29, entre la zone en treillis et la zone des fils en torons, 25. A l'endroit de la liaison entre les deux zones, des ficelles synthétiques 31 (par exemple, en PET, polyéthylène téréphtalate) encollées complètent la cohésion film/ fils en torons, lesquels pourront être eux-mêmes enrobés d'un film de protection (non représenté).

Côté distal, le film 121 est resserré à l'endroit du cerclage distal double 21, autour duquel il est retenu de part sa propre forme, ainsi que par collage et/ ou par l'intermédiaire d'une autre ficelle synthétique encollée 33.

Sur la figure 5, la vue en perspective montre mieux la forme en panier ogivale de la structure revêtue 30 avec, dans ce cas, utilisation d'un fil de commande 35 pour l'expansion radiale ou l'étirement axiale de la structure, comme expliqué avant.

La figure 6 montre que du côté proximal, les torons de fils 25 sont réunis entre eux à l'intérieur du cerclage 19 qui, à l'image du cerclage 21, peut en particulier comprendre une bague intérieure 37 et une bague extérieure 39 entre lesquelles les fils en torons sont réunis, par groupes, l'extrémité du guide-fil 5 apparaissant au centre, sa fixation pouvant être assurée par collage, soudure, ou équivalent.

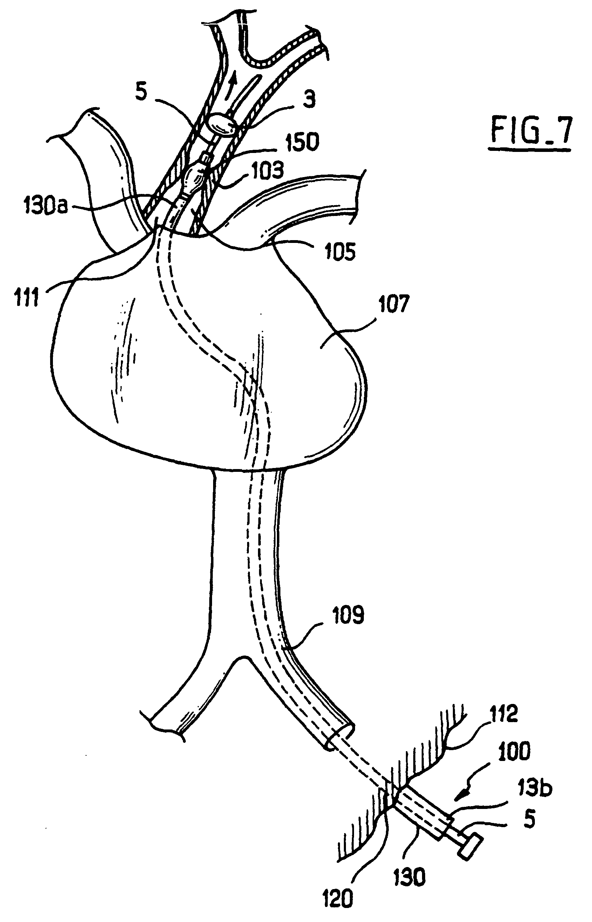

Sur la figure 7, on a repéré dans son ensemble 100 un dispositif d'angioplastie qui va permettre de réduire l'occlusion 103 du vaisseau 105.

En 107, on a repéré le coeur, en 109, l'artère aorte à travers laquelle le dispositif va être glissé, et en 111, l'ostium de l'artère carotide.

En 112 est schématisée en outre la surface cutanée du patient à travers une incision de laquelle le chirurgien va introduire le dispositif 100.

Ce dispositif comprend un premier élément allongé (cathéter) 130 extérieur équipé, vers une extrémité distale 130a d'un ballon gonflable 150 radialement déployable permettant d'écraser l'obstruction 103 afin de la réduire en vue d'améliorer les conditions de circulation du sang à l'intérieur du vaisseau.

A travers le cathéter extérieur 130 coulisse librement la tige 5 pourvue de la structure 3 (mais il pourrait s'agir de la structure 30).

Le cathéter 130 renferme deux conduits internes, l'un pour le gonflage du ballon, l'autre pour le passage de la structure distale 3.

A son extrémité proximale (hors du corps du patient), ce second conduit est raccordé à des moyens d'injection d'un produit de rinçage.

Avec un tel dispositif, une procédure de traitement peut se dérouler comme suit :

En premier lieu, le chirurgien réalise une incision cutanée, telle que l'incision 120 de la figure 7, par exemple pour réaliser une approche de l'artère carotide gauche par voie fémorale.

A travers cette incision qui conduit au vaisseau 109, le chirurgien introduit le cathéter 130.

La structure expansible distale 3 est alors dans son état radialement contraint, dans le cathéter.

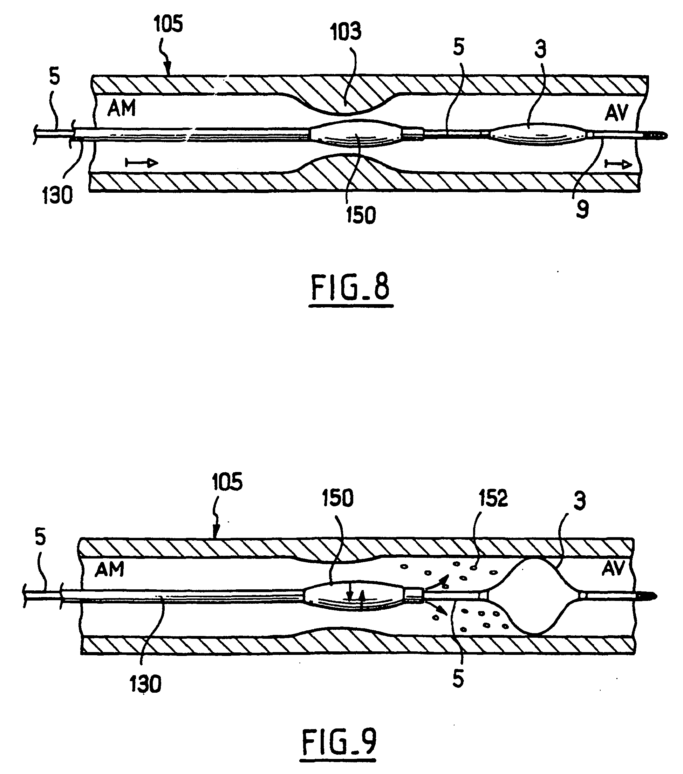

Sous conduite radiologique, elle est positionnée en aval de la sténose 103 (par rapport au sens du flux sanguin) ; voir figure 8.

On tire ensuite en arrière le cathéter 130 : la structure 3 s'expand radialement.

On peut alors placer le ballon 150 face à la sténose et le gonfler (figure 7).

A ce moment (voire un peu plus tard lors du dégonflement de l'élément 150), on injecte le produit de rinçage au travers du premier conduit du cathéter 130.

Les débris vasculaires 152 sont retenus par la structure expansée 3 (figure 9).

Une fois le rinçage effectué, l'élément 3 est resserré et le ballon dégonflé. L'ensemble du dispositif est alors retiré par la même voie que la voie d'abord, et le chirurgien suture la voie d'accès cutanée.

## Revendications

1. Dispositif vasculaire adapté pour être introduit dans un vaisseau sanguin (105) pour prévenir les embolies, en retenant d'éventuels débris vasculaires pouvant être présents dans le sang, le dispositif ayant un axe et comprenant :
- une tige (5) flexible axialement allongée,
- une structure radialement expansible (3, 3a), perméable au sang, et à laquelle la tige est liée afin qu'elle manoeuvre la structure
- la structure radialement expansible présentant axialement des extrémités proximale et distale, une première longueur suivant l'axe (1a, 1'a) et, radialement, un premier diamètre dans un état radialement resserré de la structure, et, une seconde longueur inférieure à la première et un second diamètre radial supérieur au premier diamètre dans un état radialement déployé de ladite structure, la structure ayant, suivant l'axe, une zone proximale (130a) où elle est liée à la tige, et une zone distale (130b), les zones proximale et distale s'étendant de biais par rapport à l'axe, en se rapprochant de cet axe aux extrémités proximale et distale dans ledit état radialement déployé, la structure comprenant une armature comportant un treillis de fils (30a) doublé au moins en partie par un film (121),
**caractérisé en ce qu'**il comporte une zone intermédiaire (130c) qui présente une surface tubulaire sensiblement cylindrique, où la structure présente son second diamètre dans l'état radialement déployé, et qui est de longueur supérieure à la longueur des zones proximale (130a) et distale (130b), le film pouvant laisser passer le sang sans laisser passer les débris.

2. Dispositif selon la revendication 1, **caractérisé en ce que** la zone intermédiaire est de longueur au moins double de la longueur des zones proximale (130a) et distale (130b).

3. Dispositif selon l'une des revendications 1 et 2, **caractérisé en ce que** les deux zones proximale et distale forment des surfaces sensiblement tronconiques.

4. Dispositif selon l'une des revendications précédentes, **caractérisé en ce que** le film (121) s'étend au moins sur le coté distal en y présentant plusieurs orifices (121a, 121b) destinés au passage du sang.

5. Dispositif selon l'une des revendications précédentes, **caractérisé en ce que** :
- les fils du treillis (30a) sont réunis et maintenus par des éléments de cerclage (19, 21), auxdites extrémités proximale et distale de la structure, et
- le film est fixé au moins à l'élément de cerclage distal (21).

6. Dispositif selon l'une des revendications 1 à 5, **caractérisé en ce que** la structure comporte, du côté proximal, des ouvertures (27) de taille supérieure à celle des mailles du treillis, pour laisser pénétrer dans ce treillis des débris vasculaires.

7. Dispositif selon la revendication 6, **caractérisé en ce que**, du côté proximal, mais à distance de l'extrémité proximale de la structure, les fils du treillis (30a) sont rapprochés les uns des autres par séries, pour former des torons (25) définissant entre eux lesdites ouvertures (27) de taille supérieure à celle des mailles du treillis, pour laisser pénétrer dans ce treillis des débris vasculaires.

8. Dispositif selon la revendication 7, **caractérisé en ce que** les torons de fils (25) sont réunis à l'extrémité proximale (15) de la structure où ils sont fixés à un élément de cerclage proximal (19), à l'endroit duquel la structure est en outre liée à la tige (5).

9. Dispositif selon l'une des revendications 7 et 8, **caractérisé en ce que**, du côté proximal, le film (121) s'étend sensiblement suivant le contour de la limite périphérique (29) entre la zone des fils en treillis et la zone des fils en torons.

10. Dispositif selon l'une des revendications 7 et 8, **caractérisé en ce que** :
- les torons de fils (25) s'étendent sur le côté proximal de la structure, et
- le film (121) s'étend exclusivement dans la zone intermédiaire (130c) et du côté distal (130b) de la structure, de sorte que le dispositif définit un filtre sanguin.

## Patentansprüche

1. Gefäßvorrichtung, die dazu geeignet ist, in ein Blutgefäß (105) eingeführt zu werden, um Embolien zu verhüten, wobei mögliche Gefäßteilchen zurückgehalten werden, die in dem Blut vorhanden sein können, wobei die Vorrichtung eine Achse hat und aufweist:
- einen flexiblen, axial länglichen Schaft (5),
- einen radial ausdehnbaren Aufbau (3, 3a), der für Blut durchlässig ist und mit dem der Schaft verbunden ist, damit er den Aufbau manövriert,
- wobei der radial ausdehnbare Aufbau axial proximale und distale Enden aufweist, wobei eine erste Länge der Achse (1a, 1'a) folgt und radial einen ersten Durchmesser in einem radial eingeengten Zustand des Aufbaus aufweist, und eine zweite Länge, die kleiner als die erste ist, und einen zweiten radialen Durchmesser aufweist, der in einem radial entfalteten Zustand des Aufbaus größer als der erste Durchmesser ist, wobei der Aufbau entlang der Achse eine proximale Zone (130a), wo er mit dem Schaft verbunden ist, und eine distale Zone (130b) hat, wobei sich die proximalen und distalen Zonen schräg zu der Achse erstrecken, indem sie sich dieser Achse an den proximalen und distalen Enden in dem radial entfalteten Zustand nähern und der Aufbau eine Armierung aufweist, die ein Drahtgitter (30a) enthält, das mindestens zum Teil mit einem Film (121) verkleidet ist,
- **dadurch gekennzeichnet, daß** sie eine mittlere Zone (130c) aufweist die eine im wesentlichen zylindrische, rohrförmige Oberfläche hat, wo der Aufbau seinen zweiten Durchmesser in dem radial entfalteten Zustand aufweist, und deren Länge größer ist als die Länge der proximalen (130a) und distalen (130b) Zone, wobei der Film das Blut hindurchgehen lassen kann, ohne die Teilchen hindurchgehen zu lassen.

2. Vorrichtung nach Anspruch 1, **dadurch gekennzeichnet, daß** die Zwischenzone eine Länge hat, die mindestens die doppelte Länge der proximalen (130a) und distalen (130b) Zone hat.

3. Vorrichtung nach einem der Ansprüche 1 und 2, **dadurch gekennzeichnet, daß** die beiden proximalen und distalen Zonen im wesentlichen kegelstumpfartige Oberflächen bilden.

4. Vorrichtung nach einem der vorhergehenden Ansprüche, **dadurch gekennzeichnet, daß** sich der Film (121) mindestens auf der distalen Seite erstreckt, wobei er dort mehrere Öffnung (121a, 121b) aufweist, die für den Durchgang des Blutes bestimmt sind.

5. Vorrichtung nach einem der vorhergehenden Ansprüche, **dadurch gekennzeichnet, daß**:
- die Gitterdrähte (30a) verbunden sind und durch Umreifungselemente (19, 21) an dem proximalen und distalen Ende des Aufbaus gehalten werden, und
- der Film mindestens an dem distalen Umreifungselement (21) befestigt ist.

6. Vorrichtung nach einem der Ansprüche 1 bis 5, **dadurch gekennzeichnet, daß** der Aufbau an der proximalen Seite Öffnungen (27) von größerer Abmessung als der der Gittermaschen aufweist, um in diesem Gitter Gefäßteilchen hindurchtreten zu lassen.

7. Vorrichtung nach Anspruch 6, **dadurch gekennzeichnet, daß** die Gitterdrähte (30a) an der proximalen Seite, aber im Abstand von dem proximalen Ende des Aufbaus, einander serienweise angenähert sind, um Litzen (25) zu bilden, die untereinander die Öffnungen (27) von größerer Abmessung als der der Gittermaschen bestimmen, um in diesem Gitter Gefäßteilchen hindurchgehen zu lassen.

8. Vorrichtung nach Anspruch 7, **dadurch gekennzeichnet, daß** die Drahtlitzen (25) an dem proximalen Ende (15) des Aufbaus vereinigt sind, wo sie an einem proximalen Umreifungselement (19) an der Stelle befestigt sind, wo der Aufbau außerdem mit dem Schaft (5) verbunden ist.

9. Vorrichtung nach einem der Ansprüche 7 und 8, **dadurch gekennzeichnet, daß** sich der Film (121) an der proximalen Seite im wesentlichen entlang dem Umriß der Umfangsbegrenzung (29) zwischen der Zone der Drähte als Gitter und der Zone der Drähte als Litzen erstreckt.

10. Vorrichtung nach einem der Ansprüche 7 und 8, **dadurch gekennzeichnet, daß**:
- sich die Drahtlitzen (25) auf der proximalen Seite des Aufbaus erstrecken, und
- sich der Film (121) ausschließlich in der Zwischenzone (130c) und an der distalen Seite (130b) des Aufbaus derart erstreckt, daß die Vorrichtung einen Blutfilter bestimmt.

## Claims

1. Vascular device which is suitable for being introduced into a blood vessel (105) in order to prevent embolisms by retaining any vascular debris which may be present in the blood, the device having an axis and comprising:
- an axially elongate flexible rod (5),
- a radially expandable structure (3, 3a) which is permeable to blood and to which the rod is connected in order to manoeuvre the structure,
- the radially expandable structure axially having proximal and distal ends, a first length along the axis (1a, 1'a) and, radially, a first diameter when the structure is in a radially contracted state, and a second length which is smaller than the first and a second radial diameter which is greater than the first diameter when said structure is in a radially deployed state, the structure having, along the axis, a proximal region (130a) in which it is connected to the rod, and a distal region (130b), the proximal and distal regions extending in an angled manner relative to the axis, becoming closer to this axis at the proximal and distal ends in said radially deployed state, the structure comprising a sheath which comprises a trellis (30a) of wires which is at least partially lined with a film (121),
**characterised in that** it comprises an intermediate region (130c) which has a substantially cylindrical tubular surface at which the structure has the second diameter thereof in the radially deployed state and which has a length which is greater than the length of the proximal region (130a) and distal region (130b), the film being able to allow blood to pass through without allowing the debris to pass through.

2. Device according to claim 1, **characterised in that** the intermediate region has a length which is at least double the length of the proximal region (130a) and distal region (130b).

3. Device according to either claim 1 or claim 2, **characterised in that** the two proximal and distal regions form substantially frustoconical surfaces.

4. Device according to any one of the preceding claims, **characterised in that** the film (121) extends at least over the distal side, having a plurality of openings (121a, 121b) there which are intended to allow blood to pass through.

5. Device according to any one of the preceding claims, **characterised in that**
- the wires of the trellis (30a) are joined and retained by means of annular elements (19, 21) at said proximal and distal ends of the structure, and
- the film is fixed at least to the distal annular element (21).

6. Device according to any one of claims 1 to 5, **characterised in that** the structure comprises, at the proximal side, openings (27) which are larger than the meshes of the trellis in order to allow vascular debris to be introduced into the trellis.

7. Device according to claim 6, **characterised in that**, at the proximal side, but with spacing from the proximal end of the structure, the wires of the trellis (30a) are brought together in series in order to form strands (25) which together define said openings (27) which are larger than the meshes of the trellis in order to allow vascular debris to be introduced into the trellis.

8. Device according to claim 7, **characterised in that** the strands of wires (25) are joined at the proximal end (15) of the structure where they are fixed to a proximal annular element (19), at the location of which the structure is further connected to the rod (5).

9. Device according to either claim 7 or claim 8, **characterised in that**, at the proximal side, the film (121) extends substantially along the contour of the peripheral limit (29) between the region of the wires in the form of a trellis and the region of the wires in the form of strands.

10. Device according to either claim 7 or claim 8, **characterised in that**
- the strands of wires (25) extend at the proximal side of the structure, and
- the film (121) extends exclusively in the intermediate region (130c) and at the distal side (130b) of the structure so that the device forms a blood filter.
